**Europäisches Patentamt**

**European Patent Office**

**Office européen des brevets**

(19)

(11) Publication number: **0 036 698**
**A1**

## EUROPEAN PATENT APPLICATION

(12)

(21) Application number: 81200322.6

(22) Date of filing: 23.03.81

(51) Int. Cl.³: **A 61 K 7/032**

(30) Priority: 25.03.80 NL 8001753

(43) Date of publication of application: 30.09.81
Bulletin 81/39

(84) Designated Contracting States: **AT BE CH DE FR GB IT
LI LU NL SE**

(71) Applicant: Van Buuren, Cornelis, Vlielandseweg 73,
NL-2641 KA Pijnacker (NL)

(72) Inventor: Van Buuren, Cornelis, Vlielandseweg 73,
NL-2641 KA Pijnacker (NL)

(74) Representative: Morel, Christiaan Frederik,
MORELPATENT Laarstraat 3, NL-8166 GR Emst (Epe)
(NL)

(54) **Composition of make-up for eyes.**

(57) Composition of make-up for eyes, containing an
inorganic binding-agent such as a calcium sulfate com-
pound, which after being wetted by a polar wetting
agent to a pourable suspension grows stiff or dries up
to a coherent mass.

EP 0 036 698 A1

- 1 -

Composition of make-up for eyes

The invention relates to a composition of make-up for eyes or eye-shadow and a method to make a compact make-up powder for eyes.

Generally known is a little container or box in which a disk of a certain quantity of compact eye-shadow powder is fixed and which powder is used by ladies to change their colour of the skin near their eyes. This disks of compact eye-shadow powder is manufactured by mixing a certain quantity of eye-shadow powder with a certain quantity of a binding agent first and then to compress under a high pressure, 32 - 35 atmosphere, this mixture to a disk or compact. As binding agents for example are used: micro-cellulose, dextrin (starch-gum) or carboxyl-methyl-cellulose. The obtained disks are fixed in little containers and covered by a transparant lid.

The aim of the invention is a composition of make-up for eyes or eye-shadow and a method to make a compact of make-up powder for eyes, where the use of high pressure is no longer needed to obtain a compact eye-make-up powder.

According to the invention the composition of the make-up for eyes comprises an inorganic binding agent; in the preferred composition the binding agent used is a calcium sulfate compound, such as $CaSO_4.2H_2O$, $CaSO_4.\frac{1}{2}H_2O$, etc. Through wetting the composition by a polar wetting agent, such as water, the composition becomes a pourable suspension or slurry which after pouring dries up and grows stiff so that a coherent mass is formed. The pourable suspension for example can poured directly into the little containers of boxes by means of a filling-machine; after filling, the little containers can be covered by a transparant lid. After the slurry is grown stiff it is directly ready for use. If desired the coherent mass can be slightly compressed so to improve the aspect of the final product.

Hereinafter will be described the method to make a coherent compact or disk of eye-make-up powder or eye-shadow. A certain quantity of eye-make-up powder is mixed with a certain quantity of an inorganic binding agent; then this mixture is wetted by a polar wetting agent, for example water, untill a slurry or pourable suspension is obtained and finally a number of little containers or boxes are filled with a desired quantity of this slurry. After the slurry has grown stiff or dried up the obtained product can be slightly compressed.

Also as a binding agent can be used: plaster (gypsum-cement), calcinated products of mineral mixtures of which the greater part consists of calcium sulfate or also products obtained by burning stones or rockets.

Ground gypsum ($CaSO_4.2H_2O$) can serve as a substance of eye-make-up powder. By using a weighed quantity of plaster ($CaSO_4.\frac{1}{2}H_2O$) as a binding agent there is no longer the need to use the afore mentioned binding agents such as micro-cellulose. In the method according to the invention there is no longer the obligation to compress the eye-make-up powder by means of high pressure to obtain a compact disk of the make-up powder. A great economic benefit is yield, due to the fact that the eye-make-up powder mixture can be poured directly into the little containers; no longer the disks have to be fixed into the containers; the disks of powder also won't fall out of the containers what happened once in a while with the disks made under high pressure. A special advantage of the method of manufacturing according to the invention are the unlimited possibilities to shape the thus obtained compact eye-make-up powder.

Example of a pourable slurry of an eye-make-up powder according to the invention is:

| Plaster | 10 parts |
|---|---|
| Pigment | 2 parts |
| Titaniumdioxyde | 10 parts |
| Timica | 30 parts |
| Viscous oil | 15 parts |
| Water | 55 parts |

- 1 -
CLAIMS

1. Composition of make-up for eyes, characterized in, that the composition contains an inorganic binding-agent.

2. Composition according to claim 1, characterized in, that the inorganic binding agent is a calcium sulfate compound.

3. Composition according to one of the claims 1 or 2, characterized in, that the composition is wetted by a polar wetting agent to a pourable suspension or slurry.

4. Composition according to one of the claims 1, 2 or 3, characterized in, that the composition has grown stiff or dried up to a coherent mass.

5. Composition according to one of the claims 1, 2, 3 or 4, characterized in, that the composition contains plaster (gypsum-cements) and/or calcinated products of mineral mixtures of which the greater part consists of calcium sulfate.

6. Composition according to one of the claims 1, 2, 3, 4 or 5, characterized in, that the composition contains as an inorganic binding agent a product obtained by burning stones or rockets.

7. Method to make a coherent mass of eye-make-up powder, characterized in, that a certain quantity of an inorganic binding agent is added to a certain quantity of eye-make-up powder and that next this mixture is wetted by a polar wetting agent, for example water, to a pourable suspension or slurry.

8.    Method according to claim 7, characterized in, that after the slurry has been poured for example into small containers and the slurry has grown stiff or dried up, the obtained product is slightly compressed.

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | <u>US - A - 3 800 034</u> (KIRCHER et al.)<br>* Column 1, line 39 - column 4, line 62; column 6, example 6; claims 1-4,6-7 * | 1,3,4,<br>6,7 | A 61 K 7/032 |
| | -- | | |
| X | <u>DE - B - 2 540 877</u> (SCHWAN-BLEISTIFT-FABRIK)<br>* The whole document *<br>& GB - A - 1 519 373 | 1,3,4,<br>6,7 | |
| | -- | | **TECHNICAL FIELDS SEARCHED (Int. Cl.³)** |
| X | F. WINTER: "Handbuch der gesamten Parfümerie und Kosmetik", 1927, Axel Springer Verlag, Wien, AT pages 618-624.<br>* Pages 619-624 * | 1-7 | A 61 K 7/021<br>7/031<br>7/032<br>7/035 |
| | -- | | |
| X | <u>US - A - 1 968 475</u> (BECKWITH et al.)<br>* The whole document, in particular page 1, lines 96-100 * | 1-7 | |
| | -- | | |
| X | <u>FR - A - 451 512</u> (MONIN)<br>* The whole document * | 1-3,<br>5,7 | CATEGORY OF CITED DOCUMENTS |
| | -- | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | <u>FR - A - 603 263</u> (PERL)<br>* The whole document * | 1,2,5,<br>6 | |
| | -- | | |
| | ./. | | &: member of the same patent family, corresponding document |

| The present search report has been drawn up for all claims | | |
|---|---|---|
| Place of search<br>The Hague | Date of completion of the search<br>19-06-1981 | Examiner<br>BENZ |

EPO Form 1503.1   06.78

European Patent
Office

**EUROPEAN SEARCH REPORT**

0036698

Application number

EP 81 20 0322

-2-

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | FR - A - 972 465 (GENEVAY et al.)<br>* The whole document * | 1,2,5,<br>6 | |
| | -- | | |
| X | FR - A - 504 050 (FRACHEBOND)<br>* The whole document * | 1,2,5,<br>6 | |
| | ------- | | |

TECHNICAL FIELDS
SEARCHED (Int. Cl.³)